# EUROPÄISCHE PATENTSCHRIFT

(11) **EP 1 833 424 B1**
(45) Veröffentlichungstag und Bekanntmachung des Hinweises auf die Patenterteilung: **23.07.2008**
(21) Anmeldenummer: 05815255.4
(22) Anmeldetag: 07.12.2005
(51) Int. Cl.: A61F 2/18

(54) **GEHÖRKNÖCHELCHENPROTHESE**
OSSICULAR PROSTHESIS
PROTHESE DE LA CHAINE DES OSSELETS

(30) Priorität: 09.03.2005 DE 102005010705
(43) Veröffentlichungstag der Anmeldung: 19.09.2007
(73) Patentinhaber: Heinz Kurz GmbH Medizintechnik, 72144 Dusslingen (DE)
(72) Erfinder: STEINHARDT, Uwe, 72145 Hirrlingen (DE); KURZ, Heinz, 72144 Dusslingen (DE)
(74) Vertreter: Kohler Schmid Möbus
(86) Internationale Anmeldenummer: PCT/EP2005/013077
(87) Internationale Veröffentlichungsnummer: WO 2006/094543

(56) Entgegenhaltungen:
- DE-A1- 10 045 158
- DE-U1- 20 310 609
- DE-U1- 29 904 770

## Beschreibung

Die Erfindung betrifft eine Gehörknöchelchenprothese, die mindestens ein Glied der menschlichen Gehörknöchelchenkette ersetzt oder überbrückt, wobei die Gehörknöchelchenprothese an ihren beiden Enden ein erstes und ein zweites Befestigungselement zur mechanischen Verbindung mit einem Glied der Gehörknöchelchenkette, dem Trommelfell oder dem Innenohr sowie zwischen den beiden Befestigungselementen ein Kugelgelenk aufweist, welches zwei Stege umfasst, die mit dem ersten Befestigungselement fest verbunden sind, parallel oder unter einem Winkel zueinander verlaufen und einen spaltförmigen Raum zwischen sich einschließen, in dem eine Kugel in zwei Ausnehmungen der Stege gelenkig gelagert ist, wobei die Kugel Teil eines länglichen Schafts ist, der die beiden Befestigungselemente miteinander verbindet.

Eine derartige Vorrichtung ist bekannt aus der DE 203 10 609 U1 oder aus der EP 1 181 907 B1.

Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Ambossfortsatz der menschlichen Gehörknöchelchenkette befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration durch das Trommelfell die Folge wäre. Aus diesem Grund ist es sehr hilfreich, wenn sich die Kopfplatte postoperativ selbstständig der Position des Trommelfells angleicht. Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen. Um diese Flexibilität/Variabilität zu erreichen sind verschiedene Befestigungs- und Ankopplungsvorrichtungen für Gehörknöchelchen, die elastische Teile und/oder Gelenke aufweisen, bekannt. Eine solche gelenkige Verbindung zwischen einem an der Steigbügelfußplatte montierbaren Befestigungselement und dem länglichen Schaft ist in der oben genannten EP 1 181 907 B1 beschrieben und wird von der Anmelderin unter dem Markennamen "Ball-Joint" angeboten.

Aufgrund der anatomisch- und abstammungsbedingten Variationsbreite werden im Rahmen einer Tympanoplastik unterschiedlich lange Mittelohrprothesen für die Rekonstruktion der Gehörknöchelchenkette in der Otologie benötigt. Leider ist es derzeit vor einer Operation nicht möglich, die tatsächlich erforderliche Länge zu wissen. Deshalb muss man entweder verschieden lange Prothesen am Lager haben, was mit Kosten verbunden ist, oder die Prothese muss in ihrer Länge variabel sein, so dass sie unmittelbar vor ihrer Implantation individuell auf den jeweiligen Patienten abgestimmt werden kann. Da auch die relative Lage der Gehörknöchelchenprothese zum Trommelfell bei jedem Menschen etwas differiert, muss das Mittelohr vor dem Einsatz der Prothese hinsichtlich der Lage der Befestigungselemente genau vermessen werden. Die erforderliche Länge des Schaftes kann aber erst während der Operation bestimmt werden, so dass also für jede Operation entweder ein Satz von Prothesen unterschiedlicher Länge oder eine Prothese mit variabler Schaftlänge bereitgestellt werden muss.

Eine günstige Lösung, bei der das Ablängen des Schaftes auf die individuelle Länge mit einem geringen Fertigungsaufwand für den Schaft und damit kostengünstiger folgen kann, ist beispielsweise in der EP 0 998 884 B1 beschrieben. Derartige Gehörknöchelchenprothesen werden von der Anmelderin unter dem Markennamen "VARIO" angeboten. Bei diesen bekannten Prothesen ragt der Schaft durch eine Durchgangs-bohrung des als Kopfplatte ausgebildeten ersten Befestigungselements hindurch. Der Schaft kann durch die Durchgangsbohrung axial so verschoben werden, dass er auf der Außenseite der Kopfplatte übersteht und dort abgelängt werden kann, wobei die Durchgangs-bohrung hinterher verengbar ist, um den Schaft an der gewünschten Stelle zu fixieren. Eine ähnliche Technik hinsichtlich der Ablängung ist auch in der US 6,168,625 B1 sowie der zugehörigen DE 100 45 158 A1 beschrieben, wobei hier der Schaft längs seiner Achse eine Vielzahl von Einkerbungen aufweist, die als Sollbruchstellen für die spätere unproblematische Ablängung während der Operation dienen sollen. Der dadurch entstehende wellenförmige Schaft soll eine erhöhte Friktion innerhalb eines Aufnahmeteils bewirken. Ein post-operativer automatischer Ausgleich von am Trommelfell angreifenden hydrostatischen Kräften ist aber hier schon deswegen nicht möglich, weil das Aufnahmeteil mit dem von ihm umfassten Abschnitt des wellenförmigen Schaftes kein Kugelgelenk bildet.

Als nächstliegender Stand der Technik wird DE 203 10 609 U1 angesehen.

Aufgabe der vorliegenden Erfindung ist es demgegenüber, eine gattungsgemäße Vorrichtung der eingangs beschriebenen Art dahin gehend zu verbessern, dass sowohl eine unaufwändig und kostengünstig herstellbare Gelenkstelle zur Erzielung der erforderlichen postoperativen Flexibilität und Variabilität der Prothese als auch ein mit einfachen technischen Mitteln und daher ebenfalls kostengünstig auf eine individuelle Länge einstellbarer Schaft vorhanden sind, ohne dass verschieden lange Prothesen mit hohem Kostenaufwand auf Lager gehalten oder aufwändige Spezialwerkzeuge zur individuellen Längeneinstellung während der operativen Implantation der Prothese eingesetzt werden müssen.

Erfindungsgemäß wird diese Aufgabe auf ebenso überraschend einfache wie wirkungsvolle Art und Weise dadurch gelöst, dass der längliche Schaft eine Vielzahl von aneinander angrenzenden Kugeln umfasst, von denen eine die Kugel im Kugelgelenk ist, dass der längliche Schaft durch den spaltförmigen Raum zwischen den zwei Stegen des Kugelgelenks hindurch in einer Richtung senkrecht zu den Stegen und auf das erste Befestigungselement hin oder von diesen weg und durch eine Durchbrechung des ersten Befestigungselements hindurch verschiebbar ist, wobei jeweils eine der Kugeln in einer Einrastposition zwischen den Ausnehmungen der Stege einrastet, so dass eine gewünschte Länge des Schafts modulo dem Abstand der Kugeln voneinander eingestellt und der durch das erste Befestigungselement hindurchragende, überstehende Teil des Schafts abgelängt werden kann, und dass der spaltförmige Raum zwischen den zwei Stegen des Kugelgelenks zur Fixierung des Schafts nach Einstellung der gewünschten Länge verengbar ist.

Bei der erfindungsgemäßen Gehörknöchelchenprothese sind Schaft und Kugelgelenk also keine separaten Teilelemente der Prothese, wie etwa in der Prothese nach der EP 1 181 907 B1, sondern durch den Aufbau des Schaftes als "Kugelkette" wirkt der Schaft selbst als Teil des Kugelgelenks. Dadurch werden auf wunderbar simple Weise die Vorteile der oben beschriebenen Prothese "Ball-Joint" mit denen der Prothese "VARIO" gemäß der EP 0 998 884 B1 verbunden, wobei durch die Verschmelzung von Gelenkkugel und Schaft zu einem einzigen Bauteil die ganze Vorrichtung auch noch kompakter und damit kostengünstiger herstellbar wird. Sehr wichtig ist auch die selbstständige postoperative Krafteinstellung im Lager des Kugelgeländes. Zum einen darf sie nicht zu hoch sein, denn dann findet keine Bewegung statt; zum anderen darf sie nicht zu niedrig sein, denn dies könnte zu einer fehlerhaften Signalübertragung führen. Mit der erfindungsgemäßen Vorrichtung wird auch hierfür eine optimale Lösung bereitgestellt.

Besonders kompakt und daher preiswert herstellbar ist eine Ausführungsform der erfindungsgemäßen Gehörknöchelchenprothese, bei der die beiden Stege des Kugelgelenks einstückig mit dem ersten Befestigungselement ausgeführt sind.

Eine weitere bevorzugte Ausführungsform der Erfindung umfasst eine Gehörknöchelchenprothese, bei der jeder der beiden Stege des Kugelgelenks mindestens eine, vorzugsweise mehrere nebeneinander angeordnete Ausnehmungen zur Aufnahme einer Kugel des länglichen Schafts aufweist, wobei sich immer jeweils zwei Ausnehmungen der beiden Stege gegenüberliegen. Dadurch wird während der Operation auch die Lage des Schaftes in Relation zum ersten Befestigungs-element, das in der Regel als Kopfplatte ausgebildet sein wird, in gewissen Grenzen veränderbar. Auf diese Weise kann insbesondere eine Krafteinstellung im Lager des Kugelgelenks erfolgen, so dass die Kopfplatte sich in ihrer Position postoperativ selbstständig optimal an das Trommelfell angleicht.

Eine vorteilhafte Weiterbildung dieser Ausführungsform sieht vor, dass die Ausnehmungen die Form von Rundlöchern aufweisen, was technisch besonders einfach realisierbar ist.

Bei einer alternativen Ausführungsform der Erfindung ist vorgesehen, dass jeder der beiden Stege des Kugelgelenks mindestens eine langlochförmige Ausnehmung zur in Längsrichtung des Langlochs verschiebbaren Aufnahme einer Kugel des länglichen Schafts aufweist, wobei sich immer jeweils zwei Ausnehmungen der beiden Stege gegenüberliegen. Auch damit lässt sich eine Querverschiebung des länglichen Schaftes in einer Richtung quer zu seiner Achse zwischen den beiden Stegen realisieren, um eine optimale Lageanpassung zu erreichen.

Besonders einfach und preiswert in der Herstellung ist eine Ausführungsform der erfindungsgemäßen Gehörknöchelchenprothese, bei der die Kugeln des länglichen Schafts jeweils den gleichen Außendurchmesser aufweisen und äquidistant längs der Achse des Schafts angeordnet sind. Dies erleichtert auch die Handhabung beim Ablängen des Schaftes während der operativen Implantation.

Vorteilhaft für die Herstellung ist auch eine Ausführungsform, bei der der längliche Schaft ein Stabelement umfasst, auf welches mit Durchgangsbohrungen versehene und anschließend auf dem Stabelement fixierte Kugeln aufgeschoben sind.

Besonders günstig für die Herstellung dieser Ausführungsform ist es, wenn die Kugeln mit dem Stabelement verschweißt sind, vorzugsweise mittels Laserverschweißung.

Eine vorteilhafte Weiterbildung dieser Ausführungsform sieht vor, dass auch die Durchgangsbohrungen der Kugeln mittels Laser hergestellt sind.

Ganz besonders bevorzugt ist eine Weiterbildung der oben beschriebenen Ausführungsform der erfindungsgemäßen Vorrichtung, bei welcher das Stabelement aus einem flexiblen Material gefertigt ist, was die oben beschriebene postoperative, selbstständige optimale Lageanpassung des Implantats erheblich verbessert.

Weitere bevorzugte Ausführungsformen der erfindungsgemäßen Vorrichtung zeichnen sich dadurch aus, dass die Befestigungselemente plattenförmig, glockenförmig, stempelförmig oder als Clip ausgebildet sind. Prinzipiell sind auch andere Ausbildungen der Befestigungs-elemente denkbar, jedoch haben sich die oben genannten Formen in der Praxis besonders bewährt.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. Bei vielen Ausführungsformen kann beispielsweise das erste Befestigungselement eine zur Anlage am Trommelfell ausgebildete Kopfplatte umfassen.

Andere Ausgestaltungen können vorsehen, dass die Prothese einerseits am Ambossfortsatz und andererseits am Steigbügel befestigt ist oder direkt ins Innenohr getaucht wird.

Bei wieder anderen Ausbildungen der Erfindung ist die Prothese einerseits am Hammergriff und andererseits am Amboss oder am Steigbügel befestigt ist oder wird direkt ins Innenohr getaucht.

Vorteilhaft ist in diesem Zusammenhang eine Weiterbildung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine weitere besonders bevorzugte Ausführungsform der erfindungsgemäßen Vorrichtung zeichnet sich dadurch aus, dass die Gehörknöchelchenprothese mittels Eröffnung der menschlichen Hörschnecke (=Cochleotomie) einenends direkt an das Innenohr angekoppelt ist, insbesondere über einen Kolben.

Bevorzugt ist eine Ausführungsform der erfindungsgemäßen Vorrichtung, bei der die Prothese oder Teile davon aus biokompatiblen Kunststoffen, insbesondere Silikon, oder Faserverbundwerkstoffen hergestellt ist. Dadurch können postoperative Abstoßungsreaktionen in den meisten Fällen verhindert werden.

Die erfindungsgemäße Gehörknöchelchenprothese oder Teile davon können aus Titan und/oder aus Gold und/oder aus Tantal und/oder aus einer Legierung der genannten Metalle hergestellt sein.

Vorteilhaft im Hinblick auf die oben erwähnte postoperative Lageanpassung sind Ausführungsformen der Erfindung, bei denen die Prothese oder Teile davon aus einem Material mit Formgedächtnis (=memory effect), insbesondere aus Nitinol hergestellt sind.

Ganz besonders bevorzugt ist eine Ausführungsform der erfindungsgemäßen Vorrichtung, bei der die Massenverteilung der einzelnen Teile der Prothese in Abhängigkeit von einem gewünschten, vorgebbaren Frequenzgang der Schallleitung im Mittelohr berechnet ist. Damit lässt sich gewissermaßen ein Tuning der Schallfortpflanzungs-eigenschaften mittels einer individuellen ausgestalteten Gehörknöchelchenprothese erreichen.

Ein solcher Tuning Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt.

Bei vorteilhaften Weiterbildungen dieser Ausführungsformen ist die zusätzliche Masse mittels eines Clips an einem Teil der Gehörknöchekhenkette oder der Prothese befestigt.

Eine weitere Ausführungsform der Erfindung schließlich zeichnet sich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:

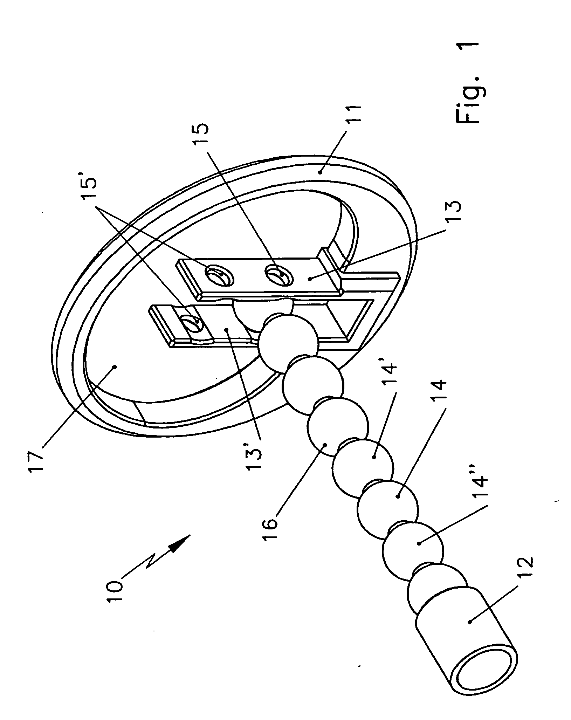

- Fig. 1: eine schematische räumliche Darstellung einer Ausführungsform der erfindungsgemäßen Vorrichtung mit einem als Kopfplatte ausgebildeten ersten Befestigungselement sowie einem stempelförmigen zweiten Befestigungselement am anderen Ende des länglichen Schaftes, wobei der längliche Schaft mit den Kugeln noch nicht in den spaltförmigen Raum zwischen den beiden Stegen eingeschoben ist; und

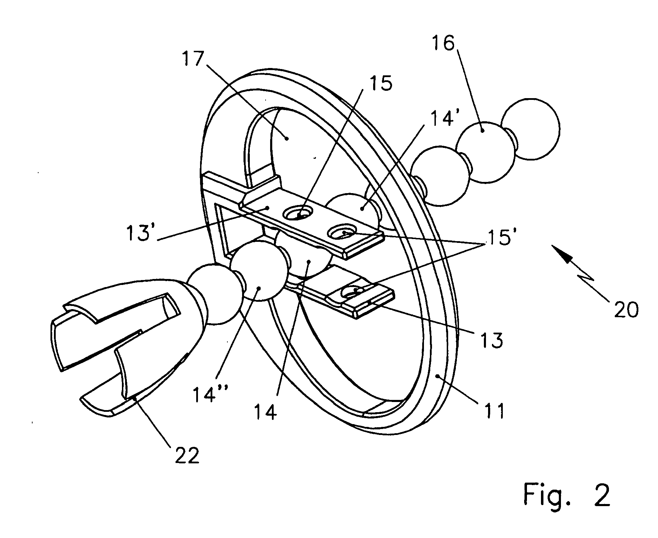

- Fig. 2: eine weitere Ausführungsform mit einem glockenförmigen statt einem stempelförmigen zweiten Befestigungselement, wobei der längliche Schaft mit einer Kugel bereits in der Einrastposition zwischen den Ausnehmungen der Stege eingerastet ist.

Die in Fig. 1 dargestellte Gehörknöchelchenprothese 10 weist ein erstes Befestigungselement 11 auf, welches in Form einer Kopfplatte zur Anlage am Trommelfell ausgebildet ist. Am anderen Ende der Gehörknöchelchenprothese 10 ist ein zweites Befestigungselement 12 vorgesehen, das bei dem vorliegenden Ausführungsbeispiel stempelförmig ausgebildet ist. Das erste Befestigungselement 11 umfasst zwei gegenüberliegende Stege 13,13', die zwischen sich einen spaltförmigen Raum einschließen und jeweils zwei nebeneinander angeordnete, im vorliegenden Ausführungsbeispiel als Rundlöcher ausgeführte Ausnehmungen 15,15' aufweisen. In diesen spaltförmigen Raum wird ein länglicher Schaft 16 eingeschoben, der eine Vielzahl von aneinander angrenzenden Kugeln 14,14',14" umfasst, und an dessen dem ersten Befestigungselement 11 abgewandten Ende das zweite Befestigungselement 12 angebracht ist.

Zur Einstellung der gewünschten Schaftlänge wird der Schaft 16 solange in axialer Richtung durch den Spalt zwischen den beiden Stegen 13,13' und einer dafür vorgesehenen Durchbrechung 17 des ersten Befestigungselements 11 hindurch geschoben, bis in der gewünschten Position eine Kugel 14 zwischen den beiden Ausnehmungen 15 einrastet. Die beiden Stege 13,13', die Kugel 14 sowie die beiden Ausnehmungen 15 bilden dann zusammen ein Kugelgelenk.

Diese Situation ist in Fig. 2 dargestellt, wobei hier allerdings das zweite Befestigungselement 22 der Gehörknöchelchenprothese 20 glockenförmig ausgebildet ist. Sobald die Kugel 14 in den beiden gegenüberliegenden Ausnehmungen 15 der Stege 13,13' eingerastet ist, kann der Schaft 16 zwischen der eingerasteten Kugel 14 und der daran angrenzenden Kugeln 14', beispielsweise mittels einer Schneidzange oder einem anderen geeigneten Mikroinstrument auf die gewünschte Endlänge abgeschnitten werden.

Die relative Position des Schaftes 16 zum ersten Befestigungselement 11 kann dadurch variiert werden, dass die Kugel 14 statt zwischen den beiden Ausnehmungen 15 parallel versetzt zwischen den beiden Ausnehmungen 15' der Stege 13,13' positioniert wird.

## Patentansprüche

1. Gehörknöchelchenprothese (10; 20), die mindestens ein Glied der menschlichen Gehörknöchelchenkette ersetzt oder überbrückt, wobei die Gehörknöchelchenprothese (10; 20) an ihren beiden Enden ein erstes (11) und ein zweites Befestigungselement (12; 22) zur mechanischen Verbindung mit einem Glied der Gehörknöchelchenkette, dem Trommelfell oder dem Innenohr sowie zwischen den beiden Befestigungselementen (11,12; 22) ein Kugelgelenk aufweist, welches zwei Stege (13, 13') umfasst, die mit dem ersten Befestigungselement (11) fest verbunden sind, parallel oder unter einem Winkel zueinander verlaufen und einen spaltförmigen Raum zwischen sich einschließen, in dem eine Kugel (14) in zwei Ausnehmungen (15) der Stege (13, 13') gelenkig gelagert ist, wobei die Kugel (14) Teil eines länglichen Schafts (16) ist, der die beiden Befestigungselemente (11,12; 22) miteinander verbindet,
**dadurch gekennzeichnet,**
**dass** der längliche Schaft (16) eine Vielzahl von aneinander angrenzenden Kugeln (14, 14', 14") umfasst, von denen eine die Kugel (14) im Kugelgelenk ist,
**dass** der längliche Schaft (16) durch den spaltförmigen Raum zwischen den zwei Stegen (13, 13') des Kugelgelenks hindurch in einer Richtung senkrecht zu den Stegen (13, 13') und auf das erste Befestigungselement (11) hin oder von diesen weg und durch eine Durchbrechung (17) des ersten Befestigungselements (11) hindurch verschiebbar ist, wobei jeweils eine der Kugeln (14, 14', 14") in einer Einrastposition zwischen den Ausnehmungen (15) der Stege (13, 13') einrastet, so dass eine gewünschte Länge des Schafts (16) modulo dem Abstand der Kugeln (14, 14', 14") voneinander eingestellt und der durch das erste Befestigungselement (11) hindurchragende, überstehende Teil des Schafts (16) abgelängt werden kann,
und **dass** der spaltförmige Raum zwischen den zwei Stegen (13, 13') des Kugelgelenks zur Fixierung des Schafts (16) nach Einstellung der gewünschten Länge verengbar ist.

2. Gehörknöchelchenprothese nach Anspruch 1, **dadurch gekennzeichnet, dass** die beiden Stege (13, 13') des Kugelgelenks einstückig mit dem ersten Befestigungselement (11) ausgeführt sind.

3. Gehörknöchelchenprothese nach einem der vorhergehenden Ansprüche, **dadurch gekennzeichnet, dass** jeder der beiden Stege (13, 13') des Kugelgelenks mindestens eine, vorzugsweise mehrere nebeneinander angeordnete Ausnehmungen (15, 15') zur Aufnahme einer Kugel (14) des länglichen Schafts (16) aufweist, wobei sich immer jeweils zwei Ausnehmungen (15 bzw. 15') der beiden Stege (13, 13') gegenüberliegen.

4. Gehörknöchelchenprothese nach Anspruch 3, **dadurch gekennzeichnet, dass** die Ausnehmungen (15, 15') die Form von Rundlöchern aufweisen.

5. Gehörknöchelchenprothese nach einem der Ansprüche 1 oder 2, **dadurch gekennzeichnet, dass** jeder der beiden Stege des Kugelgelenks mindestens eine langlochförmige Ausnehmung zur in Längsrichtung des Langlochs verschiebbaren Aufnahme einer Kugel (14) des länglichen Schafts (16) aufweist, wobei sich immer jeweils zwei Ausnehmungen der beiden Stege gegenüberliegen.

6. Gehörknöchelchenprothese nach einem der vorhergehenden Ansprüche, **dadurch gekennzeichnet, dass** die Kugeln (14, 14', 14") des länglichen Schafts (16) jeweils den gleichen Außendurchmesser aufweisen und äquidistant längs der Achse des Schafts (16) angeordnet sind.

7. Gehörknöchelchenprothese nach einem der vorhergehenden Ansprüche, **dadurch gekennzeichnet, dass** der längliche Schaft (16) ein Stabelement umfasst, auf welches mit Durchgangsbohrungen versehene und anschließend auf dem Stabelement fixierte Kugeln (14, 14', 14") aufgeschoben sind.

8. Gehörknöchelchenprothese nach Anspruch 7, **dadurch gekennzeichnet, dass** die Kugeln (14, 14', 14") mit dem Stabelement verschweißt sind, vorzugsweise mittels Laserverschweißung.

9. Gehörknöchelchenprothese nach einem der Ansprüche 7 oder 8, **dadurch gekennzeichnet, dass** die Durchgangsbohrungen der Kugeln (14, 14', 14") mittels Laser hergestellt sind.

10. Gehörknöchelchenprothese nach einem der vorhergehenden Ansprüche, **dadurch gekennzeichnet, dass** die Befestigungselemente (11,12; 22) plattenförmig, glockenförmig, stempelförmig oder als Clip ausgebildet sind.

11. Gehörknöchelchenprothese nach einem der vorhergehenden Ansprüche, **dadurch gekennzeichnet, dass** das erste Befestigungselement (11) eine zur Anlage am Trommelfell ausgebildete Kopfplatte umfasst.

12. Gehörknöchelchenprothese nach einem der vorhergehenden Ansprüche, **dadurch gekennzeichnet, dass** die Prothese oder Teile davon aus Titan und/oder aus Gold und/oder aus Tantal und/oder aus einer Legierung der genannten Metalle hergestellt ist.

13. Gehörknöchelchenprothese nach einem der vorhergehenden Ansprüche, **dadurch gekennzeichnet, dass** die Prothese oder Teile davon aus einem Material mit Formgedächtnis, was gleichbedeutend mit memory effect ist, insbesondere aus Nitinol hergestellt ist.

## Claims

1. An auditory ossicle prosthesis (10; 20) which replaces or bridges at least one link of the human auditory ossicle chain, wherein the auditory ossicle prosthesis (10; 20) has at its both ends a first (11) and a second fastening element (12; 22) for mechanical connection to a link of the auditory ossicle chain, the eardrum or the inner ear, and has between the two fastening elements (11, 12; 22) a ball joint which comprises two ridges (13, 13') which are fixedly connected to the first fastening element (11), run parallel or an angle to each other and enclose between them a gap-shaped space in which a ball (14) is mounted in articulated fashion in two recesses (15) of the bridges (13, 13'), wherein the ball (14) is part of an oblong shaft (16), which connects the two fastening elements (11, 12; 22) together,
**characterised in that**
the longitudinal shaft (16) comprises a multiplicity of adjacent balls (14, 1', 14"), one of which is the ball (14) in the ball joint,
**in that** the oblong shaft (116) is displaceable through the gap-shaped space between the two bridges (13, 13') of the ball joint in a direction perpendicular to the bridges (13, 13') and towards the first fastening element (11), or away from this and through an opening(17) of the first fastening element (11), wherein each of the balls (14, 14', 14") engages in an engaging position between the recesses (14) of the bridges (13, 13') so that a desired length of the shaft (16) can be set according to the distance of the balls (14, 14', 14") from each other and so that the part of the shaft (16) projecting through the first fastening element (11) can be cut to length, and **in that** the gap-shaped space between the two bridges (13, 13') of the ball joint can be narrowed for fixing the shaft (16) after adjusting the desired length.

2. The auditory ossicle prosthesis according to Claim 1, **characterised in that** the two bridges (13, 13') of the ball joint are designed integrally with the first fastening element (11).

3. The auditory ossicle prosthesis according to one of the preceding claims, **characterised in that** each of the two bridges (13, 13') of the ball joint has at least one, preferably a plurality of adjacently arranged recesses (15, 15') for receiving a ball (14) of the oblong shaft (16), wherein two recesses (15 and 15') of the two bridges (13, 13') always lie opposite each other.

4. The auditory ossicle prosthesis according to Claim 3, **characterised in that** the recesses (15, 15') have the shape of round holes.

5. The auditory ossicle prosthesis according to one of Claims 1 or 2, **characterised in that** each of the two bridges of the ball joint has at least one slot-shaped recess for receiving, displaceably in the longitudinal direction of the slot, a ball (14) of the oblong shaft (16), wherein two recesses of the two bridges always lie opposite each other.

6. The auditory ossicle prosthesis according to one of the preceding claims, **characterised in that** the balls (14, 14', 14") of the oblong shaft (16) each have the same outside diameter and are arranged equidistantly along the axis of the shaft (16).

7. The auditory ossicle prosthesis according to one of the preceding claims, **characterised in that** the oblong shaft (16) comprises a bar element onto which are pushed balls (14, 14', 14") which are provided with through bores and are then fixed on the bar element.

8. The auditory ossicle prosthesis according to Claim 7, **characterised in that** the balls (14, 14', 14") are welded to the bar element, preferably by means of laser welding.

9. The auditory ossicle prosthesis according to one of Claims 7 or 8, **characterised in that** the through bores of the balls (14, 14', 14") are produced by means of a laser.

10. The auditory ossicle prosthesis according to one of the preceding claims, **characterised in that** the fastening elements (11, 12; 22) are designed in the shape of a plate, bell, piston or as a clip.

11. The auditory ossicle prosthesis according to one of the preceding claims, **characterised in that** the first fastening element (11) comprises a head plate designed to bear against the eardrum.

12. The auditory ossicle prosthesis according to one of the preceding claims, **characterised in that** the prosthesis or parts thereof is/are produced from titanium and/or from gold and/or from tantalum and /or from an alloy of the metals mentioned.

13. The auditory ossicle prosthesis according to one of the preceding claims, **characterised in that** the prosthesis or parts thereof is/are produced from a material with shape memory, which is synonymous with memory effect, in particular from nitinol.

## Revendications

1. Prothèse d'osselet de l'oreille (10 ; 20) qui remplace ou couvre au moins un élément de la chaîne des osselets humaine, la prothèse d'osselet de l'oreille (10 ; 20) présentant au niveau de ses deux extrémités un premier (11) et un second élément de fixation (12 ; 22) pour la liaison mécanique avec un élément de la chaîne des osselets, le tympan ou l'oreille interne ainsi qu'entre les deux éléments de fixation (11, 12 ; 22), une articulation sphérique qui comprend deux parties de liaison (13, 13') qui sont reliées solidement au premier élément de fixation (11), s'étendent parallèlement ou selon un angle l'une par rapport à l'autre et contiennent un espace en forme de fente entre elles, dans lequel une sphère (14) est logée de manière articulée dans deux évidements (15) des parties de liaison (13, 13'), la sphère (14) faisant partie d'une tige (16) oblongue qui relie les deux éléments de fixation (11, 12 ; 22),
**caractérisée en ce**
**que** la tige oblongue (16) comprend une pluralité de sphères (14, 14', 14") contiguës, dont l'une est la sphère (14) de l'articulation sphérique,
en ce que la tige oblongue (16) peut être déplacée au travers de l'espace en forme de fente entre les deux parties de liaison (13, 13') de l'articulation sphérique dans une direction perpendiculaire aux parties de liaison (13, 13') et vers le premier élément de fixation (11) ou loin de celui-ci et au travers d'un percement (17) du premier élément de fixation (11), respectivement l'une des sphères (14, 14', 14") s'encliquetant dans une position encliquetée entre les évidements (15) des parties de liaison (13, 13') de sorte qu'une longueur souhaitée de la tige (16) puisse être réglée en modulant la distance des sphères (14, 14', 14") entre elles et la partie de la tige (16) dépassant, pénétrant dans le premier élément de fixation (11) puisse être mise à longueur, et en ce que l'espace en forme de fente entre les deux parties de liaison (13, 13') de l'articulation sphérique peut être contracté pour la fixation de la tige (16) après le réglage de la longueur souhaitée.

2. Prothèse d'osselet de l'oreille selon la revendication 1, **caractérisée en ce que** les deux parties de liaison (13, 13') de l'articulation sphérique sont réalisées en une seule pièce avec le premier élément de fixation (11).

3. Prothèse d'osselet de l'oreille selon l'une des revendications précédentes, **caractérisée en ce que** chacune des deux parties de liaison (13, 13') de l'articulation sphérique présente au moins un, de préférence plusieurs évidements (15, 15') disposés l'un à côté de l'autre pour recevoir une sphère (14) de la tige (16) oblongue, respectivement deux évidements (15 ou 15') des deux parties de liaison (13, 13') se faisant toujours face.

4. Prothèse d'osselet de l'oreille selon la revendication 3, **caractérisée en ce que** les évidements (15, 15') présentent la forme de trous ronds.

5. Prothèse d'osselet de l'oreille selon l'une des revendications 1 ou 2, **caractérisée en ce que** chacune des deux parties de liaison de l'articulation sphérique présente au moins un évidement en forme de trou oblong pour la réception mobile dans le sens longitudinal du trou oblong d'une sphère (14) de la tige oblongue (16), deux évidements des deux parties de liaison se faisant toujours face respectivement.

6. Prothèse d'osselet de l'oreille selon l'une des revendications précédentes, **caractérisée en ce que** les sphères (14, 14', 14") de la tige oblongue (16) présentent chacune le même diamètre extérieur et sont disposées à équidistance le long de l'axe de la tige (16).

7. Prothèse d'osselet de l'oreille selon l'une des revendications précédentes, **caractérisée en ce que** la tige oblongue (16) comprend un élément en forme de barrette, sur lequel sont enfilées des sphères (14, 14', 14") pourvues de perçages traversants et fixées ensuite sur l'élément en forme de barrette.

8. Prothèse d'osselet de l'oreille selon la revendication 7, **caractérisée en ce que** les sphères (14, 14', 14") sont soudées à l'élément en forme de barrette, de préférence en utilisant une soudure au laser.

9. Prothèse d'osselet de l'oreille selon l'une des revendications 7 ou 8, **caractérisée en ce que** les perçages traversants des sphères (14, 14', 14") sont ménagés au moyen du laser.

10. Prothèse d'osselet de l'oreille selon l'une des revendications précédentes, **caractérisée en ce que** les éléments de fixation (11, 12 ; 22) sont réalisés en forme de plaque, de cloche, de vérin ou comme un clip.

11. Prothèse d'osselet de l'oreille selon l'une des revendications précédentes, **caractérisée en ce que** le premier élément de fixation (11) comprend une plaque de recouvrement réalisée pour l'appui sur le tympan.

12. Prothèse d'osselet de l'oreille selon l'une des revendications précédentes, **caractérisée en ce que** la prothèse ou des parties de celle-ci sont fabriquées en titane et/ou en or et/ou en tantale et/ou en un alliage des métaux cités.

13. Prothèse d'osselet de l'oreille selon l'une quelconque des revendications précédentes, **caractérisée en ce que** la prothèse ou des parties de celle-ci sont fabriquées en un matériau à mémoire de forme, ce qui est synonyme de memory effect, en particulier en nitinol.
